# EUROPÄISCHE PATENTSCHRIFT

(11) **EP 0 616 006 B1**
(45) Veröffentlichungstag und Bekanntmachung des Hinweises auf die Patenterteilung: **11.06.1997**
(21) Anmeldenummer: 94103471.2
(22) Anmeldetag: 08.03.1994
(51) Int. Cl.: C08J 11/06, A61L 9/01

(54) **Verfahren zur Desodorierung gebrauchter Schaumstoff-Formteile, insbesondere von Fischkisten**
Process for deodorising used foamed mouldings, especially crates of fish
Procédé pour désodoriser des pièces moulés de mousse usés, en particulier de caisses de poissons

(30) Priorität: 17.03.1993 DE 4308430
(43) Veröffentlichungstag der Anmeldung: 21.09.1994
(73) Patentinhaber: BASF Aktiengesellschaft, 67063 Ludwigshafen (DE)
(72) Erfinder: Witt, Michael, Dr., D-67071 Ludwigshafen (DE); Scherzer, Dietrich, Dr., D-67433 Neustadt (DE); Hahn, Klaus, Dr., D-67281 Kirchheim (DE); Henn, Rolf, Dr., D-68775 Ketsch (DE); Back, Wolf-Dieter, Dr., D-67434 Neustadt (DE)

(56) Entgegenhaltungen:
- EP-A- 0 252 695
- DATABASE WPI Derwent Publications Ltd., London, GB; AN 84-130968[21] & JP-A-59 066 500 (LION CORP.) 14. April 1984
- DATABASE WPI Derwent Publications Ltd., London, GB; AN 92-145117[18] & JP-A-4 077 534 (NISSAN MOTOR KK.) 11. März 1992
- DATABASE WPI Derwent Publications Ltd., London, GB; AN 84-097722[16] & JP-A-59 043 037 (TOYO RUBBER CHEM. IND. KK.) 9. März 1984
- DATABASE WPI Derwent Publications Ltd., London, GB; AN 90-012612[02] & JP-A-1 293 872 (MITSUBISHI GAS CHEM. KK.) 27. November 1989
- DATABASE WPI Derwent Publications Ltd., London, GB; AN 84-130968[21] & JP-A-59 066 500
- DATABASE WPI Derwent Publications Ltd., London, GB; AN 92-145117[18] & JP-A-4 077 534
- DATABASE WPI Derwent Publications Ltd., London, GB; AN 84-097722[16] & JP-A-59 043 037
- DATABASE WPI Derwent Publications Ltd., London, GB; AN 90-012612[02] & JP-A-1 293 872

## Beschreibung

Die Erfindung betrifft ein Verfahren zur Desodorierung gebrauchter Schaumstoff-Formteile, insbesondere von Flschkisten.

Fischkisten aus expandierbarem Polystyrol (EPS) können nicht direkt der Wiederverwertung zugeführt werden, da der typische Fischgeruch (hauptsächlich verursacht durch Trimethylamin) durch ein thermisches Aufschmelzen in konventionellen Extrudern nicht zu entfernen ist. Viele der in der Literatur beschriebenen Maßnahmen zur Beseitigung dieses Geruchsproblems haben den Nachteil, daß die geruchsbildenden Verunreinigungen nicht wirklich entfernt, sondern lediglich gebunden werden oder der unangenehme Geruch lediglich mit anderen Gerüchen überdeckt wird.

Zu erwähnen sind hier insbesondere Eisensalze in Kombination mit verschiedenen Carbonsäuren (JP-A-62/194 863), Kolchizin (JP-A-60/261 459), Cyclodextrin (JP-A-63/59 962) oder GlyoxalBisulfit-Addukte (JP-A-54/28 826).

Zur Überdeckung des Fischgeruches wurden außerdem parfümartige Essentien (Pflanzenextrakte) vorgeschlagen.

Diese Verfahren sind jedoch teuer und führen zu weiteren Beimengungen im Polymeren, die die Verarbeitung im Extruder oder die spätere Anwendung des Polymeren negativ beeinflussen können.

Auch die Reinigung mit heißem Wasser zeigte sich als wenig wirksam.

5 Aufgabe der Erfindung war es daher, ein einfaches Verfahren zur Desodorierung gebrauchter Schaumstoff-Formteile, insbesondere von Fischkisten, bereitzustellen. Dieses Verfahren sollte es ermöglichen, die geruchsbildenden Verunreinigungen möglichst quantitativ zu entfernen. Das hierdurch erhaltene gereinigte Schaumstoff-Formteil sollte dadurch entweder direkt einer erneuten Verwendung oder zumindest der stofflichen Wiederverwertung zugänglich sein.

Demgemäß wurde ein Verfahren zur Desodorierung gebrauchter Schaumstoff-Formteile gefunden, bei dem die gebrauchten Schaumstoff-Formteile mit einer wäßrigen Lösung von Phosphorsäure, wasserlöslichen primären oder sekundären Phosphaten oder ihren Mischungen, behandelt werden.

Wasserlösliche primäre oder sekundäre Phosphate sind hierbei insbesondere die entsprechenden Alkalisalze mit den Formeln M^{I}H₂PO₄ bzw. M^{I}₂HPO₄, worin M^{I} für ein Alkaliatom wie Natrium oder Kalium, insbesondere Natrium, steht.

Die hierbei einzusetzenden Konzentrationen hängen vom Verschmutzungsgrad sowie der beabsichtigten Einwirkungszeit der Lösung auf die zu reinigenden Schaumstoff-Formteile ab.

Erfindungsgemäß enthält die wäßrige Lösung Phosphorsäure, wasserlösliche primäre oder sekundäre Phosphate oder ihre Mischungen vorzugsweise in einer Menge von 0,001 bis 5,0 Gew.-%, bezogen auf die Lösung.

Die im erfindungsgemäßen Verfahren eingesetzten gebrauchten Schaumstoff-Formkörper können aus einem beliebigen Schaumstoff bestehen.

Das erfindungsgemäße Verfahren ist jedoch besonders geeignet für Partikel-Schaumstoffe auf der Basis von Styrolpolymerisaten oder Polyolefinen.

Besonders bevorzugt werden Schaumstoff-Formteile auf der Basis von Styrolpolymerisaten (Polystyrol-Partikelschaum) eingesetzt. Styrolpolymerisate sind hierbei Polystyrol oder Styrolcopolymerisate mit mindestens 50 Gew.-% einpolymerisiertem Styrol.

Die Herstellung dieser bekannten Polystyrolschaumstoff-Formkörper und der ihnen zugrundeliegenden expandierbaren Styrolpolymerisate (EPS) ist an sich bekannt (vgl. beispielsweise EP-B-0 106 129; Kunststoffe, Band 67 (1977), Seiten 617 bis 621).

Das erfindungsgmäße Verfahren ist besonders vorteilhaft bei der Desodorierung von Fischkisten, kann aber auch zur Reinigung anderweitig gebrauchter Schaumstoff-Formteile benutzt werden.

Es zeigte sich, daß durch das erfindungsgemäße Verfahren ganze gebrauchte Fischkisten gereinigt werden können. Überraschenderweise erhalten die ursprünglich weißen Fischkisten wieder ihr makelloses weißes Aussehen zurück. Dies ist für eine direkte Wiederverwertung der Fischkisten von besonderer Bedeutung.

Auch zu "Briketts" komprimierte Fischkisten oder zerkleinerte Schaumstoffteile können auf diese Weise gereinigt werden.

Erfindungsgemäß ist es von Vorteil, wenn die Schaumstoff-Formteile anschließend an die Behandlung mit einer wäßrigen Lösung von Phosphorsäure, wasserlöslichen primären oder sekundären Phosphaten oder ihren Mischungen mit Wasser behandelt werden.

Andernfalls kann beispielsweise im Falle von Fischkisten aus Polystyrol-Partikelschaum beim Aufschmelzen eine Verfärbung des Polystyrols eintreten.

### Beispiele

### Beispiel 1

Zu Briketts mit einer Dichte von 0,4 g/cm komprimierte Fischkisten wurden zunächst zu ca. 2 cm³ großen Brocken zerkleinert. 1 Gew.-Teil dieser Brocken wurde für 12 Stunden bei Raumtemperatur in 5 Gew.-Teilen einer 0,1 gew.-%igen wäßrigen Phosphorsäurelösung dispergiert. Anschließend wurde die wäßrige Phase abgetrennt, die Brocken erneut für 1 Stunde mit 5 Gew.-Teilen Wasser dispergiert und das Wasser abgetrennt. Mit Hilfe einer Presse konnte abschließend das Wasser nahezu vollständig entfernt werden.

An den Brocken konnte kein Fischgeruch mehr festgestellt werden. Nach der Extrusion konnte ein bernsteinfarbenes Polystyrolgranulat erhalten werden, das frei von jeglichem Fischgeruch war.

Wenn stattdessen nur 10 Minuten in der Phosphorsäure und (zur anschließenden Wäsche) 10 Minuten in Wasser dispergiert wurde, war noch ein geringer Fischgeruch feststellbar.

### Beispiel 2

Zur Aufbereitung mit dem Ziel einer mehrmaligen Verwendung wurde eine intakte Fischkiste während 12 Stunden mit einer 0,1 gew.-%igen Na₂HPO₄-Lösung bei Raumtemperatur besprüht.

Die Fischkiste zeigte nach dieser Behandlung eine makellose weiße Oberfläche. Fischgeruch konnte nicht mehr festgestellt werden.

Bei einer Besprühung während lediglich 15 Minuten mit einer 0,1 gew.-%igen Na₂HPO₄-Lösung bei Raumtemperatur, war die Fischkiste anschließend zwar äußerlich sauber, es konnte jedoch noch ein Fischgeruch festgestellt werden. Wenn hierbei allerdings eine 60°C warme Na₂HPO₄-Lösung eingesetzt wurde, konnte der Fischgeruch deutlich verringert werden.

## Patentansprüche

1. Verfahren zur Desodorierung gebrauchter Schaumstoff-Formteile, dadurch gekennzeichnet, daß die gebrauchten Schaumstoff-Formteile mit einer wäßrigen Lösung von Phosphorsäure, wasserlöslichen primären oder sekundären Phosphaten oder ihren Mischungen, behandelt werden.

2. Verfahren nach Anspruch 1, dadurch gekennzeichnet, daß die wäßrige Lösung Phosphorsäure, wasserlösliche primäre oder sekundäre Phosphate oder ihre Mischungen in einer Menge von 0,001 bis 5,0 Gew.-%, bezogen auf die Lösung, enthält.

3. Verfahren nach Anspruch 1, dadurch gekennzeichnet, daß die Schaumstoff-Formteile aus einem Polystyrol-Partikelschaum bestehen.

## Claims

1. A process for the deodorization of used foam moldings, which comprises treating the used foam moldings with an aqueous solution of phosphoric acid, water-soluble primary or secondary phosphates or mixtures thereof.

2. A process as claimed in claim 1, wherein the aqueous solution contains from 0.001 to 5.0% by weight, based on the solution, of phosphoric acid, water-soluble primary or secondary phosphates or mixtures thereof.

3. A process as claimed in claim 1, wherein the foam moldings comprise a polystyrene particle foam.

## Revendications

1. Procédé de désodorisation d'articles moulés en mousse ou matière expansée utilisés, caractérisé en ce que l'on traite les articles moulés en mousse ou matière expansée utilisés par une solution aqueuse d'acide phosphorique, de phosphates primaires ou secondaires hydrosolubles, ou leurs mélanges.

2. Procédé suivant la revendication 1, caractérisé en ce que la solution aqueuse contient l'acide phosphorique, les phosphates primaires ou secondaires hydrosolubles ou leurs mélanges en une proportion de 0,001 à 5,0% en poids, par rapport à la solution.

3. Procédé suivant la revendication 1, caractérisé en ce que les articles moulés en mousse ou matière expansée sont constitués d'une mousse particulaire de polystyrène.
